# EUROPEAN PATENT APPLICATION

(11) **EP 4 162 976 A1**
(43) Date of publication of application: **12.04.2023**
(21) Application number: 21201176.1
(22) Date of filing: 06.10.2021
(51) Int. Cl.: A61N 1/372, A61B 5/00

(54) **SYSTEM COMPRISING A PLURALITY IMPLANTABLE MEDICAL DEVICES AND A REMOTE COMPUTER FACILITY**

(71) Applicant: BIOTRONIK SE & Co. KG, 12359 Berlin (DE)
(72) Inventor: MÜLLER, Jens, 14195 Berlin (DE); DIEM, Björn Henrik, 14197 Berlin (DE)
(74) Representative: Biotronik Corporate Services SE

(57) **Abstract**

A system comprising a plurality of implantable medical devices (1) and a remote computer facility (10) is described, wherein the remote computer facility comprises an artificial implant program assessment service (AIPAS service, 11), wherein the AIPAS service is configured to collect, store, and process data of a plurality of patients (A to I, 36, 37) and of the plurality of medical devices, wherein processing of these data is provided using an AI module and comprises an assessment of these data, wherein the AIPAS service is further configured to provide for a specific patient and a specific medical device at least one parameter value for a patient-specific operation of the specific medical device within the body of the specific patient based on the processed data of the AIPAS service, on the data of the specific patient and on the data of the specific medical device as well as based on an assessment of at least one specific patient data and/or of at least one specific medical device data with regard to at least one benchmark data value. Further, a respective operation method of such system, a computer program product and a computer readable data carrier is described.

## Description

The invention is directed to a system comprising at least one implantable medical device and a remote computer facility and to an operation method of such system as well as to a computer program product and to a computer readable data carrier.

An implantable medical device is an active or passive implantable medical device, for example, an implant such as a pacemaker (with leads), an Implantable Cardiac Monitor (ICM), an Implantable Leadless Pacer (ILP), an Implantable Leadless Pressure Sensor (ILPS), an Implantable Cardiac Defibrillator (ICD) or a Subcutaneously Implanted Cardiac Defibrillator (S-ICD), a device that delivers spinal cord stimulation (SCS), deep brain stimulation (DBS) or neurostimulation. Such medical device may deliver one or more therapeutic substances, e.g. a drug pump, contain sensors that collect physiological signals in order to monitor the health status of the patient and/or deliver a therapy to the patient, for example using electromagnetic waves.

Today's implantable medical devices contain at least one processor that operate according to a collection of instructions that are executed by the at least one processor to perform the pre-defined task. This collection of instructions is called the program. In order to adapt the operation of one specific medical device to the specific patient which receives the specific medical device by implantation at least one pre-defined parameter value is provided to the specific medical device that is stored in this medical device and queried during execution of the internal algorithm of the medical device within the body of the patient. Usually a plurality of parameter values, e.g. a parameter value set, is used for the specific adaption of the algorithm of the medical device. Such single parameter value or set of parameter values may, for example, determine the amplitude of therapy pulses, measurement intervals, or the selection/deselection of specific program sections representing, e.g. different program options, so that the medical device optimally operates within the body of the specific patient and provides the optimal therapy. Nowadays the at least one parameter value is transmitted to the medical device, for example, after implantation and/or prior or during activation of the medical device. The determination and transmission of at least one parameter value to the medical device is called programming in the following.

Currently, health care practitioner (HCP - e.g. physician, nurse, trained technician) have to update the parameter value set for an implantable medical device manually and for each patient/medical device combination separately and individually. Further, the HCP has the possibility to view the relevant information of the medical device via the Home Monitoring Service Center (HMSC). This is possible because the medical device sends remote monitoring messages to the HMSC on a regular and event-based basis. The quality of the program adapted thereby is based on the HCP's knowledge and experience and can be very time-consuming. Further, the amount of information and data that needs to be taken into account for parameter value set creation increases steadily: the capabilities of the medical devices increase, so do the number of program options. Due to medical and technical progress, the number of available diagnostic data and vital data of each patient increases as well. Accordingly, the complexity of data assessment during follow-ups increases.

However, the main concern of any program update is to find the best program adaption for a specific implantable medical device according to the very specific medical history, condition and indication of the specific patient (amongst other patient related data). The expression "medical history, condition and indication" in the previous sentence shows that there is a multitude of different data to consider for an implantable medical device program. In general, "the best program adaption" is one that uses a set of specific, suitable parameter values in order to achieve two things: a high wellbeing and a long life of the patient treated by the specific medical device as well as optimal operation of the medical device (e.g. with regard to battery longevity).

The HCP may consider an electronic patient file/health record for adaption of at least one program parameter value, wherein the electronic patient file/health record is the entity that stores all data related to one patient. Usually, given the number of available patients and the number of available data for each patient and the number of medical device data, it is impossible for an HCP to manually analyse all these data and determine the set of parameter values that best program adaption of an implantable medical device.

Currently, at least in cardiac rhythm management (CRM) context, most medical devices can only be configured, reconfigured and programmed by means of an external programming device (so-called programmer). This applies for both implantation and follow-up. This is cost-intensive as the programming device has to be adapted to the specific needs of the specific medical device to be programmed so that for different medical device different programming devices have to be handled and serviced. This makes the clinic workflows additionally complex.

Accordingly, it is desirable to provide alternative/additional guidance for the HCP in order to improve the medical device operation, to optimize the workflow in the clinic and to reduce the costs for follow-ups.

The above problem is solved by a system with the features of claim 1 and by an operation method with the features of claim 10 as well as a computer program product with the features of claim 14 and the computer readable data carried defined in claim 15.

In particular, the above problem is solved by a system comprising a plurality of implantable medical devices and a remote computer facility, wherein the remote computer facility comprises an artificial implant program assessment service (AIPAS service), wherein the AIPAS service is configured to collect, store, and process data of a plurality of patients and of the plurality of medical devices, wherein processing of these data is provided using an AI module and comprises an assessment of these data, wherein the AIPAS service is further configured to provide for a specific patient and a specific medical device at least one parameter value for a patient-specific operation of the specific medical device within the body of the specific patient based on the processed data of the AIPAS service, on the data of the specific patient and on the data of the specific medical device as well as based on an assessment of at least one specific patient data and/or of at least one specific medical device data with regard to at least one benchmark data value.

The above system is based on the idea that the AIPAS service with its AI module collects, stores and processes data of a plurality of patients and of a plurality of medical devices by AI methods, wherein processing comprises an assessment of these data, in order to find a high quality program update of the specific medical device for the very specific medical history, condition and indication of each patient (amongst other patient related data). Thereby, the AI module is capable to use patient data and medical device data of different clinics, regions, countries or even continents and therefore has access to an exponential number of patient files and medical device files. The selection of parameter value update for the program using the AI module allows to save money for clinics and medical practices by helping to minimize time for reconfiguration/follow-up of the respective medical device. The basis for the parameter value assessment of a patient specific program forms the automatic analysis and assessment of the AI module of a large number of existing programs for different indications and patient histories. This allows to identify more efficient/successful parameter combinations. While primary therapy settings might be often the same for a larger number of patients, the change of certain parameters leads to better personalized care having higher program quality. The AIPAS service thereby provides one-click programming (i.e. confirming the proposed program parameter values only) that saves time for the HCP. Further it is no longer necessary to use an external programming device to support follow-ups of medical devices, so that the clinics save all these costs and the workflow in the clinic is optimized, as well.

The assessment of at least one specific patient data and/or of at least one specific medical device data with regard to at least one benchmark data value comprises/operates and/or the input for all calculations should be two or more of:
- Age
- Gender
- Activity level
- Medication
- Symptom:
   ∘ Cardiovascular arrest
   ∘ Syncope
   ∘ Presyncope
   ∘ Palpitations
   ∘ Congestive heart failure
   ∘ Dizzy Spells
   ∘ Cerebral Dysfunction
   ∘ Other
- ECG Indication:
   ∘ Normal sinus rhythm
   ∘ Ventricular fibrillation
   ∘ Ventricular tachycardia
   ∘ Ventricular tachycardia/fibrillation
   ∘ Complete Heart Block related
   ∘ Bundle branch block related (incl. Left and Right bundle branch block)
   ∘ Hemiblock related
   ∘ QRS widening
   ∘ Other
- Etiology:
   ∘ Coronary artery disease
   ∘ Myocardial infarction
   ∘ Conduction tissue fibrosis
   ∘ Dilative cardiomyopathy (ischemic)
   ∘ Dilative cardiomyopathy (non-ischemic)
   ∘ Arrhythmogenic RV cardiomyopathy
   ∘ Long QT syndrome
   ∘ Valvular heart disease
   ∘ Other heart disease
   ∘ Idiopathic
   ∘ Carotid Sinus Syndrome
   ∘ Vasovagal Syndrome
   ∘ Cardiomyopathy
   ∘ Congenital
   ∘ Surgical
   ∘ Ablation
   ∘ Drug related
   ∘ Heart transplantation
   ∘ Not documented
   ∘ Other
- Device Type (IPG, ICD, Nr. Of chambers, Manufacturer Information, etc.)
- Electrode Types
- Active Program (Parameter settings within implant)

Optimized parameter may be calculated for at least one of the following programs:
- Heart Failure with normal sinus function
- Heart Failure with permanent AF
- Chronotropic incompetence in Heart Failure with low patient activity
- Chronotropic incompetence in Heart Failure with moderate patient activity
- Chronotropic incompetence in Heart Failure with high patient activity
- AT/AF prevention

At least one of the following parameters may be optimized within/before mentioned programs:
∘ Mode
∘ Ventricular Detection Interval (incl. Onset and Stability)
∘ Basic Rate
∘ Rate Hysteresis
∘ Ventricular Upper Rate
∘ Atrial Upper Rate
∘ Biventricular Pacing VV Delay
∘ AV Delay after Sense (Heart Rate < 100 bpm)
∘ AV Delay after Pace (Heart Rate < 100 bpm)
∘ AV Delay after Sense (Heart Rate >= 100 bpm)
∘ AV Delay after Pace (Heart Rate >= 100 bpm)
∘ Max. Sensor Rate
∘ Capture Control Threshold
∘ Home Monitoring related parameter

In one embodiment, the AIPAS service is further configured to collect, store and process data of a scientific study and/or of a national or international health organization and to assess data of the plurality of patients and of the plurality of medical devices with regard to the data of the scientific study and/or to the data of the national or international health organization. This allows to additionally include new scientific results or suggestions/requirements of a national or international health organization into the process of parameter value determination and thereby further improve program quality.

The remote computer facility and the AIPAS service may comprise any hardware or virtual (cloud) computing facility which may include a single computer and network of computers.

The AIPAS service is a functionality of the remote computer facility realized by respective hardware and software. As indicated above, the AIPAS service is configured to collect, store and process data of a plurality of patients and of a plurality of medical devices. The AIPAS service processes the data (including filtering, pre-processing and post-processing) using at least one processor contained in the remote computer facility. For collection of data and provision of the at least one program parameter value the AIPAS service further comprises at least one respective interface and/or communication module provided by the remote computer facility. For storage of data the AIPAS service comprises a data memory which is also provided by the remote computer facility.

The data memory may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device.

The communication of the communication module with the medical device(s) and, if applicable, relay device(s) may be provided wirelessly and/or the air using electromagnetic waves, for example Medical Implant Communication Service (MICS), Bluetooth, WLAN, ZigBee, NFC, Wibree or WiMAX in the radio frequency region, or IrDA or free-space optical communication (FSO) in the infrared or optical frequency region or by wire (electrical and/or optical communication), or coil telemetry and/or Z impedance telemetry.

The at least one processor of the AIPAS service within the remote computer facility may be operated centrally (on-premises) or de-centrally on multiple hardware and software instances (hybrid or cloud-based). The at least one processor of the remote computer facility performs substantial computations, including numerous arithmetic operations and logic operations without human intervention, such as, for example, a personal mobile device (PMD), a desktop computer, a server computer, clusters/warehouse scale computer or embedded system.

In order to generate a holistic picture for therapy decisions provided by the medical device, a wide variety of health information of the patients may be used and processed by the AI module which is realized particularly by the at least one processor and the data memory of the AIPAS service. The processing of the data includes assessment of these data.

The communication module may receive various medical and non-medical information from the HCPs, the patients, clinics or any other information provider (e.g. studies of a university) sporadic or on an event-based or cyclical basis (pull and push mechanisms are possible) and stores this information in the data memory.

In one embodiment, the AIPAS service is configured to collect data of the plurality of patients from different medical domains such as cardiac rhythm management, neurology, ophthalmology, surgery, gynecology, otorhinolaryngology, dermatology, venerology, internal medicine, pediatrics, laboratory medicine, nuclear medicine, psychiatry, psychotherapy, radiology, urology but also from fields such as nutritional science. The AIPAS service also stores these data in the data memory and processes them as indicated below.

The medical device is any device as indicated above which is configured to be implanted partly or fully within a patient's body and is configured to monitor the health condition of a patient and/or to deliver a therapy to the patient. Particularly, the medical device may be, for example, any implant, a pacemaker, ILP, ICD, S-ICD, device that delivers SCD, implantable sensors e.g. pressure sensor or an ICM.

Processing of the received data is provided using the AI module. The AIPAS service is configured to provide for a specific patient and a specific medical device at least one program parameter value for a patient-specific operation of the specific medical device within the body of the specific patient based on the processed data of the AIPAS service, on the data of the specific patient and on the data of the specific medical device as well as based on an assessment of at least one specific patient data and/or of at least one specific medical device data with regard to at least one benchmark data value. The AI module uses artificial intelligence (AI) and/or machine learning (ML) methods to achieve a basis for parameter value determination.

Clustering may be one embodiment of an AI method used by the AI module to compare large numbers of complex data sets, identify (hidden) relationships and similarities within the data sets in order to get a new interpretation of the data. It identifies patients with similar patient files and/or medical device files. Clustering is provided using unidimensional clusters (i.e. a cluster that take one category or attribute into account, e.g. the age of a patient) or multidimensional clusters (i.e. cluster that take at least two categories or attributes into account, e.g. the age and the predominant cardiological indication).

The term AI module may be understood in the sense of software components or software instances which are designed to correctly interpret data, to learn from such data, and to use those learnings to provide a medical support function through flexible adaptation. The AI module may be realized by a neural network. A neural network is based on a collection of connected nodes. A node that receives a signal processes it and can signal nodes connected to it. In one embodiment, nodes are aggregated into layers. Different layers may perform different transformations on their inputs. Signals travel from an input layer to an output layer. Such a neural network may be trained by processing examples, each of which contains a known input and result, forming probability-weighted associations between the two, which are stored within the data structure of the neural network. Thus, the neural network learns to perform tasks by considering examples. To train the neural network, the deep learning method is used. For example, the neural network is a feed forward network with several hidden layers or a recurrent neural network with several hidden layers. The neural network may include input signal conditioning and post processing of the results. Optionally, the neural network may have a model governance layer to make the medical application traceable. Such a system may then either be "frozen" at the end of the training phase and used as a validated classifier or the system can be further trained with additional data from regular reporting and continuously improved in the reporting result. In this case, however, it is necessary to implement a continuous quality assurance process in parallel in the operation of the system to avoid an unwanted deviation in the wrong direction. This may be realized, for example, by regularly also performing findings with the data of the original training data set and automatically evaluating the result and resetting the system to an "older" training state in case of unacceptable deviations.

By the AI module, patient data and medical device data may be classified, for example, using categories, attributes and intervals. Each category may be a topic for at least one, for example at least two attributes, wherein each attribute refers to data of the same kind. Intervals are portions of the data values of each attribute. For example, the following classification of patient's data may be used, wherein the category is written in front of the brackets and the attributes within the brackets:
- the demographical data of the patient (attribute examples: age, gender, race, place of birth, nationality, place of living, occupation, income, details about children, education),
- the medical history of the patient (attribute examples: prior illnesses & surgeries no matter if directly related to current cardiological indication, MRI/CRT images, referral/discharge letters, psychological assessment(s))
- the cardiological indication(s) of the patient,
- the medication of the patient (attribute examples: current medication, history of medication, medication related to cardiological indication, medication related to neurological indication, medication related to other indications),
- data/history about the physical condition and/or vital data of the patient (attribute examples: walked steps, heart rate (min, max, median), sleep data), and
- patient self-assessments (attribute examples: patient diaries, patient surveys).

With regard to data of the medical device, the following classification may be used:
- medical device data (attribute examples: implant type, implant manufacturer, implantation date, lead data, current program, old program(s), reason for program changes, alerts, events, ECG/EEG data, implants test data, implants sensor data).

There is no need to say that these data categories are examples only. The larger the patient file or the higher the number of medical device information, the more data categories are available, the better the result. The same applies to the amount of data per category: the more, the better.

The AI module executes the clustering within one initial step being a learning step. If the AIPAS service receives new data, the clustering is at least partially rerun, for example in regular time intervals, in order to extend and/or improve the clustering.

Additionally, not all categories might have an equal significance and not all attributes of a category might have an equal significance. Accordingly, the AI module may apply different quantification and/or weight to different categories and/or attributes. Those significances may be permutated and the AI module of the AIPAS service may run multiple times with different quantification and/or weight of categories and attributes.

Further, the above examples use very general categories, but the method or system may be used for very specific patient or medical device data, as well. Consider patients with the already very similar indication and medication and their medical device programs differ only in a small number of parameter values. Analysing and clustering their ECG data only may result in finding a higher quality parameter value combination of the medical device program. Additionally, the output of the AIPAS service after assessment of the data may comprise at least one setting for Home Monitoring Service Centre alert.

As a result of the clustering of the AI module, in one embodiment, all patient data and medical device data are assigned to at least one cluster and each cluster is assigned to at least one single specific parameter value or to a set of specific parameter values representing the respective program. If then, for any new specific patient and specific medical device combination the at least one parameter value (usually several parameter values) for a high quality program shall be determined, in one embodiment, the AI module may search - using, for example, the clustering - for the most similar patient and medical device combination based on the specific patient data and the specific medical device data. If the AI module has found the most similar patient and medical device, the at least one parameter value (usually several parameter values) assigned to the most similar patient and medical device (e.g. the at least one parameter value of the cluster of the most similar patient and medical device) is determined as benchmark value and is compared with the recent parameter value for the actual specific patient and medical device during assessment.

The data of the specific device and the specific patient for determination of the at least one parameter value may be combined from multiple sources and may include (but are not limited to)
- data from the specific medical device, including the type of the specific medical device (result of test runs, measured parameters of body, medical device statistics, therapy success etc.), including program parameters of the past and recent program parameters,
- sensor data provided by specific patient (personal health data from smart watches e.g. symptoms, quality of life survey, disease specific questionnaires, data from wearables such as blood pressure, activity),
- data of the specific patient collected by physician at pre-implantation consulting (main indication, sub-indications, diagnosis, patient history, medication, laboratory results, other medical assessments) and included directly to remote network service, and
- data of the specific patient collected from other medical sources like EHR systems (main indication, sub-indications, diagnosis, patient history, medication, laboratory results, other medical assessments)
- relevant events concerning the patient or patient group or medical device (complaints, studies, new medical findings)
- content of a data base (e.g. of the provider of the medical device or the clinic) with best practices.

In one embodiment, the AIPAS service is configured such that the at least one parameter value for the patient-specific operation of the specific medical device is provided automatically or on request, for example by an HCP or the medical device. For example, the HCP may request the determination of the at least one parameter value for a scheduled follow-up.

In one embodiment, the AI module is configured to use an AI (artificial intelligence) method such as clustering of said data and/or Bayesian networks and/or deep learning neuronal networks. The mentioned AI methods are especially suitable for the present invention.

In one embodiment, the AIPAS service comprises a user interface and is further configured to provide the at least one parameter value for the patient-specific operation of the specific medical device for the specific patient at the user interface for confirmation and/or further adaption by an HCP and/or is configured to receive the data of the specific patient and/or the data of the specific medical device at the user interface. This means that the HCP may input the specific patient data and/or the specific medical device data using the user interface. Additionally or alternatively, the user interface provides the determined at least one parameter value for programming of the specific medical device at the user interface, for example on a display with keyboard, tablet or touchscreen. Then, the HCP may check the at least one parameter value and may afterwards confirm and/or adapt the at least one parameter value. Accordingly, the HCP has the possibility to change the at least one parameter value before it is realized in the program of the specific medical device for the specific patient, for example after checking of the at least one parameter value and/or activation of the medical device. This increases the safety in use of the specific medical device.

In another embodiment, the AIPAS service is further configured to transmit the at least one parameter value for the patient-specific operation directly without HCP intervention or after confirmation and/or adaption by the HCP to the specific medical device and/or to directly receive the data of the specific patient and/or the data of the specific medical device. For that, the AIPAS service and/or the medical device comprises a respective transmitter, transceiver and/or receiver (module). This is a very cost-effective way of transferring the at least one parameter value to the medical device and thereby programming this device. After checking the at least one parameter value by the medical device and/or activation, the device executes its program based on the transmitted at least one parameter value. In one embodiment, the AIPAS service may transmit the at least one parameter value to the specific medical device via a relay device, for example a tablet or a mobile telephone. In one embodiment, the relay device is configured to bidirectionally communicate with the medical device for receiving patient's data and/or device's data from the medical device and with the remote computer facility. The bidirectional communication may use the above-mentioned communication methods.

In one embodiment, the at least one medical device is configured such that one request for the at least one parameter value to the AIPAS service is provided by each medical device automatically during a regular follow-up. Embodiments for both possibilities are explained below. The determination of at least one parameter value during a regular follow-up may be provided in order to adapt the algorithm of the medical device with a higher accuracy and/or to changed circumstances with the patient (e.g. additional illness, increased fitness of the patient etc.).

In one embodiment, the AIPAS service may be implemented into or connected with a home monitoring service, wherein the home monitoring service provides manual or automatic monitoring of bodily measurement values and device measurement values provided by the medical device, wherein the medical device transmits these measurement values to the remote computer facility. The AIPAS service may update its data basis of the patient and/or the medical device and may update its data processing (i.e. AI analysis by the AI module) using the measurement values provided by the home monitoring.

The above problem is further solved by an operation method of a system comprising a plurality of implantable medical devices and a remote computer facility, wherein the remote computer facility comprises an artificial implant program assessment service (AIPAS service), wherein the AIPAS service collects, stores and processes data of a plurality of patients and of a plurality of medical devices, wherein processing of these data is provided using an AI module and comprises an assessment of these data, wherein the AIPAS service further provides for a specific patient and a specific medical device at least one parameter value for a patient-specific operation of the specific medical device within the body of the specific patient based on the processed data of the AIPAS service, on the data of the specific patient and of the specific medical device as well as based on an assessment of at least one specific patient data and/or of at least one specific medical device data with regard to at least one benchmark data value.

In one embodiment, the AIPAS service provides the at least one parameter value for the patient-specific operation of the specific medical device automatically or on request, for example by an HCP or the medical device.

In one embodiment, the AI module executes an AI method such as clustering of said data and/or uses the AI method of Bayesian networks and/or deep learning neuronal networks.

In one embodiment, the AIPAS service transmits the at least one parameter value for the patient-specific operation directly without HCP intervention or after confirmation and/or adaption by the HCP to the specific medical device.

The above embodiments of the operation method have the same advantages as the above system. Embodiments of the system indicated above may be realized in the operation method analogously. It is referred to the above explanation of the system in this regard.

The above method may, further, be realized as a computer program which comprises instructions which, when executed, cause the system to perform the steps of the above method which is a combination of above and below specified computer instructions and data definitions that enable computer hardware to perform computational or control functions or which is a syntactic unit that conforms to the rules of a particular programming language and that is composed of declarations and statements or instructions needed for a above and below specified function, task, or problem solution.

Furthermore, the above defined method may be part of a computer program product comprising instructions which, when executed by a processor, cause the processor to perform the steps of the above defined method. Accordingly, a computer readable data carrier storing such computer program product is disclosed.

The present invention will now be described in further detail with reference to the accompanying schematic drawing, wherein
- Fig. 1: shows a first embodiment of the inventive system;
- Fig. 2: depicts a second embodiment of the inventive system;
- Fig. 3: shows steps of the inventive operation mode of the inventive system; and
- Fig. 4: depicts examples of clustering provided by the AI module of the inventive system.

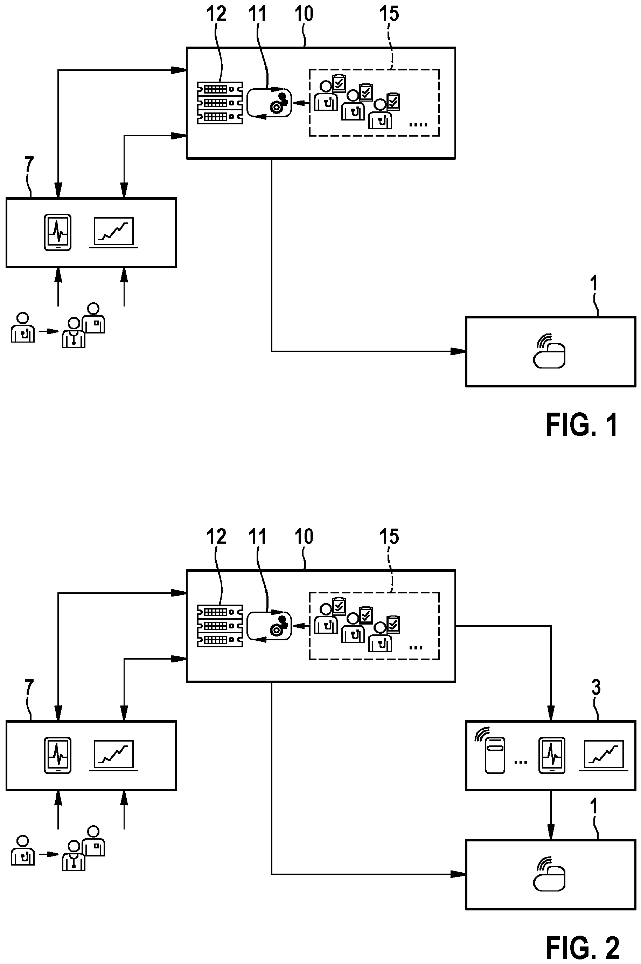

The first embodiment of the inventive systems shown in Fig. 1 comprises the medical device in form of a pacemaker 1 and the remote computer facility 10. The remote computer facility comprises an AIPAS service 11, a plurality of computers 12 forming a network and a data memory 15. The system further may comprise a device 7 providing the user interface for the remote computer facility, for example a tablet, touchscreen or display with a keyboard. The second embodiment of the system shown in Fig. 2 additionally comprises a relay device 3, e.g. a mobile telephone for communication of the remote computer facility 10 and medical device 1.

The AIPAS service 11 comprises or consists of at least one state-of-the-art computer or workstation (with state-of-the-art calculation power an/or access to remote calculation power, e.g. via cloud computing) that uses an AI module providing an artificial intelligence (AI) and/or machine learning (ML) method, i.e. the AIPAS service consists of a combination of hardware and software to realize the described functionality. For example, the AIPAS service 11 comprises the AI module that is sketched in the following providing machine learning (in general) and unsupervised learning and clustering (in specific).

Within the remote computer facility 10, the AIPAS service 11, the plurality of computers 12 and the data memory 15 are electrically and/or optically connected in order to exchange data. The data memory 15 is realized as indicated above in the general part of the description. Each computer/workstation comprises at least one processor.

The device 7, the remote computer facility 10 (and its components), and the medical device 1 are configured such that they bidirectionally communicate data with each other. The data communication is realized by using at least one of the above communication methods. If a relay device 3 is used as depicted in Fig. 2 there is an at least one-directional communication connection using one of the above methods of the remote computer facility 10 and the relay device 3 as well as the relay device 3 and the medical device 1.

The AI module of the AIPAS service 11 uses the AI method of clustering to compare large numbers of complex data sets of patient data and medical device data, identify (hidden) relationships and similarities within the data sets and help to get a new interpretation of these data. It is used to identify patients with similar patient files and then subsequently suggesting an existing implant program for a (different) new patient.

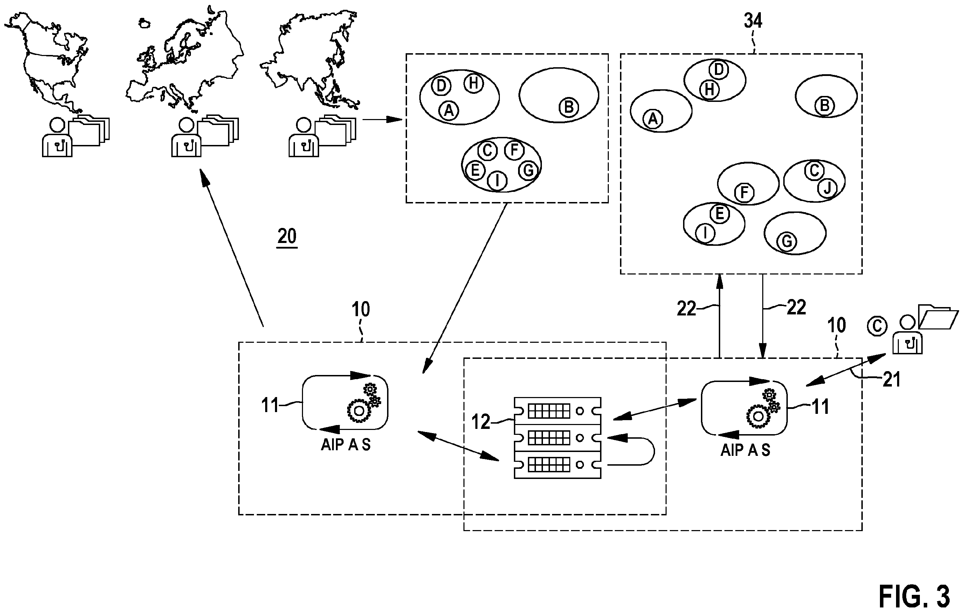

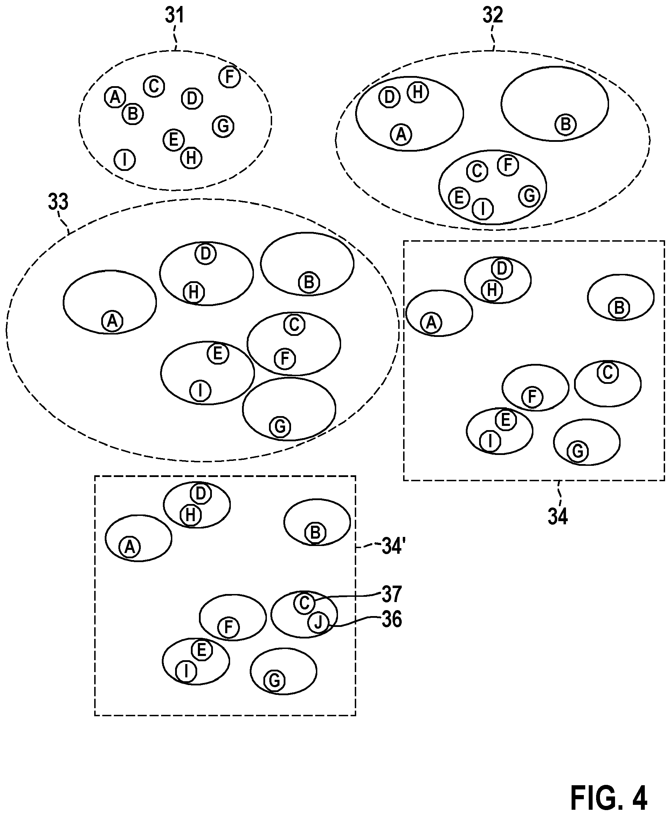

Clustering is provided by the AI module in an initial learning step of the AIPAS service which is represented by the loop with reference sign 20 in Fig. 3 is explained with regard to an example of 9 patients (see patients A to I in section 31 of Fig. 4, each with a simple patient file consisting of the following data categories
- the demographical data of the patient (consisting of the single attribute age),
- the current cardiological indication, and
- the current medication.

Further, to each patient A to I in section 31 before clustering, a respective individual parameter set of a medical device is known.

The AI algorithm of the AI module creates in the initial learning step 20 from the data clusters of similar data, e.g. patients with same age belong to the same cluster. Definition of clusters are not fixed and may be intelligently changed by the algorithm. Considering the attribute age, the cluster definition may be, for example:
10-year-interval: age 0-10, age 11-20, age 21-30 and so on or
5-year-interval: age 0-5, age 6-10, age 11-15, age 16-20 and so on or
1-year-interval: age 0, age 1, age 2, age 3 and so on.

A unidimensional clustering considering the attribute age, for example, is shown in section 32 of Fig. 4. It comprises three clusters of a 10-year-interval of age, each containing 1 to 5 patients (see small circles). The AI module, in principle, may provide such unidimensional clustering but the strength of clustering using AI methods consists in multidimensional clustering.

During initial training 20 provided by the AI module it starts with the aforementioned clustering for age (unidimensional), then the method takes other attributes into account and alters the cluster composition with it. For example, in section 33 of Fig. 4 it is shown a two-dimensional clustering considering age in a 10-year-interval and three different current cardiological indications forming 6 clusters with one or two patients, whereas section 34 of Fig. 4 shows a tree-dimensional clustering having 7 clusters with regard to the attributes age in the 10-year-interval, three different current cardiological indications and three different medications as another example, wherein each cluster contains 1 to 2 patients. These clusters are multidimensional and give a well-founded basis for providing any new specific medical device for a specific patient with program parameter values. Clustering may consider medical device data as well. To each of the clusters a set of parameter values is assigned which is particular suitable for patients and medical devices having the properties defined for this cluster.

With that multidimensional clustering as a result, after taking all data (i.e. all available patient data from all clinics, all countries, all continents and each patient file with all available data categories with all attributes and all available medical device data) into account, the system is trained.

If a specific patient 36 with specific patient data is scheduled to visit the HCP during a follow-up for his/her a specific implanted medical device, e.g. a pacemaker, the specific patient data, the specific medical data (e.g. the indication) and data from the specific medical device (e.g. sensor measurements, most recent program parameter set) are transmitted to the remote computer facility 10 with AIPAS service 11, for example, triggered by the responsible HCP. Additionally or alternatively, these or similar data may be transmitted from the HMSC. This is represented by arrow 21 on the right-hand side of Fig. 3. The AIPAS services 11 analyses the specific patent data and specific medical device data based on its current clustering revealed by training (see clustering of section 34' in Fig. 4 that is similar to the clustering of section 34) and identifies the most similar patient (by using its trained AI module) which is denoted in clustering 34' with reference number 37 (see arrows 22). Accordingly, the AIPAS service 11 assigns the new patient 36 into the cluster of the identified most similar patient 37. Based on this analysis the AIPAS service 11 determines the set of parameter values that is assigned to this cluster (cluster with patient 37) as the benchmark for the program parameters of the specific medical device. The AIPAS service 11 then compares the most recent program parameters with the program parameters of this cluster and/or with guidelines for program parameters relating to this cluster and determines whether at least one program parameter needs to be changed (updated/adapted) or not. The AIPAS service then transmits the changed set of parameters or the updated parameters to the responsible HCP (see arrow 21 in Fig. 3) and suggests this set of parameter values as program for this specific patient and specific medical device to the HCP at the device 7 (e.g. a notebook) forming a user interface. After the HCP has confirmed the suggested set of parameter values or the adapted parameter values these data are transmitted by the AIPAS service to the medical device 1 either directly (see Fig. 1) or via a relay device 3 (e.g. a tablet or smartphone, see Fig. 2). The transmitted set of parameter values or the adapted parameter values is/are then checked in the medical device 1. After successful checking by the medical device 1 the medical device executes its program based on the transmitted set of parameters / adapted parameter values.

If the HCP does not (fully) agree with the proposed parameter set for the combination of specific patient/specific medical device, they change the proposed parameter value set or adapted parameter values and confirms the changed data. It is then transmitted to the medical device 1 and checked as explained above.

If the check within the medical device 1 revealed that a problem occurred, the medical device 1 informs the HCP, for example using the AIPAS service 11 and the device 7 with the user interface.

In another embodiment, the patient data transmitted by the HCP to the AIPAS service comprise medication data and/or data from any medical device sensor.

Alternatively (which may be configured with the remote computer facility 10) the determined new program parameter values or changed set of program parameter values for the specific patient and specific medical device are transmitted automatically to the medical device 1 and checked (as soon as communication is available) without HCP confirmation.

In the embodiments explained above the remote computer facility 10, in particular its AIPAS service, may remind (by alarm) the HCP that a new program parameter value suggestion is available.

Additionally, with regard to both embodiments, determined program parameter values for the specific patient and specific medical device are transmitted via common interface and protocol to program the specific medical device during implantation procedure and after implantation procedure from remote computer facility 10 (directly or indirectly via relay 3) without the need of a separate external programming device.

In one embodiment, the data determined and transmitted by the AIPAS service 11 to the responsible HCP may comprise at least one setting for Home Monitoring Service Center alert assigned to the cluster of the specific patient 36.

The above system and method optimize the workflow in the clinic and administration because an external programming device is not necessary as well as no development, production, logistics and maintenance costs for such external programming device. Further therapy quality and individual care is increased by added computational effort. By assessing the present program parameter values and automatically determining adapted program parameter values, the HCP is unburdened and the error-prone manual assessment and adaption of a medical device program for each specific patient is replaced by a review of the artificially created medical device program.

Accordingly, the above system and method pioneer the topic of workflow optimization in the clinic and digital health. They save a lot of money by omitting an external programming device and by minimizing the time for programming. Further, therapy quality and individual care are increased.

## Claims

1. A system comprising a plurality of implantable medical devices (1) and a remote computer facility (10), wherein the remote computer facility comprises an artificial implant program assessment service (AIPAS service, 11), wherein the AIPAS service is configured to collect, store, and process data of a plurality of patients (A to I, 36, 37) and of the plurality of medical devices, wherein processing of these data is provided using an AI module and comprises an assessment of these data, wherein the AIPAS service is further configured to provide for a specific patient and a specific medical device at least one parameter value for a patient-specific operation of the specific medical device within the body of the specific patient based on the processed data of the AIPAS service, on the data of the specific patient and on the data of the specific medical device as well as based on an assessment of at least one specific patient data and/or of at least one specific medical device data with regard to at least one benchmark data value.

2. The system of claim 1, wherein the AIPAS service is further configured to collect, store and process data of a scientific study and/or of a national or international health organization and to assess data of the plurality of patients and of the plurality of medical devices with regard to the data of the scientific study and/or to the data of the national or international health organization.

3. The system of any one of the previous claims, wherein the AIPAS service is configured such that the at least one parameter value for the patient-specific operation of the specific medical device is provided automatically or on request, for example by an HCP or the medical device.

4. The system of any one of the previous claims, wherein the AI module is configured to use an AI method such as clustering of said data and/or Bayesian networks and/or deep learning neuronal networks.

5. The system of any of the previous claims, wherein the AIPAS service is further configured to provide the at least one parameter value for the patient-specific operation of the specific medical device for the specific patient at a user interface for confirmation and/or further adaption by an HCP and/or to receive the data of the specific patient and/or the data of the specific medical device at the user interface.

6. The system of any of the claims 1 to 4, wherein the AIPAS service is further configured to transmit the at least one parameter value for the patient-specific operation directly without HCP intervention or after confirmation and/or adaption by the HCP to the specific medical device and/or to directly receive the data of the specific patient and/or the data of the specific medical device.

7. The system of any of the claims 3 to 6, wherein the at least one medical device is configured such that one request for the at least one parameter value to the AIPAS service is provided by each medical device automatically during a regular follow-up.

8. The system of any of the previous claims, wherein the AIPAS service is configured such that for any new specific patient and specific medical device combination it searches for the patient data and/or medical device data already processed by the AIPAS service most similar to the patient data and/or medical device data of the new patient.

9. The system of any of the previous claims, wherein the AIPAS service is further configured to transmit the at least one parameter value to the specific medical device via a relay device (3).

10. An operation method of a system comprising a plurality of implantable medical devices (1) and a remote computer facility (10), wherein the remote computer facility comprises an artificial implant program assessment service (AIPAS service, 11), wherein the AIPAS service collects, stores and processes data of a plurality of patients (A to I, 36, 37) and of a plurality of medical devices, wherein processing of these data is provided using an AI module and comprises an assessment of these data, wherein the AIPAS service further provides for a specific patient and a specific medical device at least one parameter value for a patient-specific operation of the specific medical device within the body of the specific patient based on the processed data of the AIPAS service, on the data of the specific patient and of the specific medical device as well as based on an assessment of at least one specific patient data and/or of at least one specific medical device data with regard to at least one benchmark data value.

11. The method of claim 10, wherein the AIPAS service provides the at least one parameter value for the patient-specific operation of the specific medical device automatically or on request, for example by an HCP or the medical device.

12. The method of any one of the claims 10 to 11, wherein the AI module executes an AI method such as clustering of said data and/or uses the AI method of Bayesian networks and/or deep learning neuronal networks.

13. The method of any one of the claims 10 to 12, wherein the AIPAS service transmits the at least one parameter value for the patient-specific operation directly without HCP intervention or after confirmation and/or adaption by the HCP to the specific medical device.

14. A computer program product comprising instructions which, when executed by a processor, causes the processor to perform the steps of the method according to any of the claims 10 to 13.

15. Computer readable data carrier storing a computer program product according to claim 14.
